# EUROPEAN PATENT SPECIFICATION

(11) **EP 1 649 289 B1**
(45) Date of publication and mention of the grant of the patent: **22.10.2008**
(21) Application number: 04741160.8
(22) Date of filing: 20.07.2004
(51) Int. Cl.: G01N 33/574

(54) **USE OF PROTEINS PROTEINASE 3 (PRN3) AND LEUKOCYTE ELASTASE INHIBITOR (ILEU) AS A MARKER FOR COLORECTAL CANCER**
VERWENDUNG DER PROTEINE PROTEINASE 3 (PRN3) UND LEUKOZYTEN ELASTATSE INHIBITOR (ILEU) ALS MARKER FÜR KOLOREKTALE KARZINOME
UTILISATION DE LA PROTEINASE 3(PRN3) ET DE L'INHIBITEUR DE L'ELASTASE LEUCOCYTAIRE (ILEU) EN TANT QUE MARQUEUR POUR LE CANCER COLORECTAL

(30) Priority: 21.07.2003 EP 03015695
(43) Date of publication of application: 26.04.2006
(73) Proprietor: Roche Diagnostics GmbH, 68305 Mannheim (DE); F. Hoffmann-La Roche AG, 4070 Basel (CH)
(72) Inventor: TACKE, Michael, 81373 Muenchen (DE); BERNDT, Peter, CH-4056 Basel (CH); HAGMANN, Marie-Luise, 82377 Penzberg (DE); KARL, Johann, 82380 Peissenberg (DE); LANGEN, Hanno, 79585 Steinen (DE); PALME, Stefan, 82377 Penzberg (DE); ROESSLER, Markus, 82110 Germering (DE); ROLLINGER, Wolfgang, 82398 Polling (DE); ZOLG, Werner, 82362 Weilheim (DE)
(86) International application number: PCT/EP2004/008083
(87) International publication number: WO 2005/015219

(56) References cited:
- MOLLDREM J ET AL: "TARGETED T-CELL THERAPY FOR HUMAN LEUKEMIA: CYTOTOXIC T LYMPHOCYTESSPECIFIC FOR A PEPTIDE DERIVED FROM PROTEINASE 3 PREFERENTIALLY LYSE HUMAN MYELOID LEUKEMIA CELLS" BLOOD, W.B.SAUNDERS COMPAGNY, ORLANDO, FL, US, vol. 88, no. 7, 1 October 1996 (1996-10-01), pages 2450-2457, XP000884740 ISSN: 0006-4971
- MOLLDREM JEFFREY J ET AL: "Cytotoxic T lymphocytes specific for a nonpolymorphic proteinase 3 peptide preferentially inhibit chronic myeloid leukemia colony-forming units" BLOOD, vol. 90, no. 7, 1997, pages 2529-2534, XP002300135 ISSN: 0006-4971
- DAVID A.; KACHER Y.; SPECKS U.; AVIRAM I.: 'Interaction of proteinase 3 with CD11b/CD18 (beta2 integrin) on the cell membrane of human neutrophils.' JOURNAL OF LEUKOCYTE BIOLOGY vol. 74, no. 4, 15 July 2003, pages 551 - 557

### SEQUENCE LISTING

<110> Roche Diagnostics GmbH F. Hoffmann-La Roche AG
<120> Use of PRN3/ILEU as a marker for colorectal cancer
<130> 21821
<150> EP 03015695.4
   <151> 2003-07-21
<160> 2
<170> PatentIn version 3.2
<210> 1
   <211> 256
   <212> PRT
   <213> Homo sapiens
<220>
   <221> MISC_FEATURE
   <223> Leukocyte proteinase 3, synonymous with Myeloblastin [Precursor]
<400> 1
   Met Ala His Arg Pro Pro Ser Pro Ala Leu Ala Ser Val Leu Leu Ala
   1               5                   10                  15
   Leu Leu Leu Ser Gly Ala Ala Arg Ala Ala Glu Ile Val Gly Gly His
               20                  25                  30
   Glu Ala Gln Pro His Ser Arg Pro Tyr Met Ala Ser Leu Gln Met Arg
           35                  40                  45
   Gly Asn Pro Gly Ser His Phe Cys Gly Gly Thr Leu Ile His Pro Ser
       50                  55                  60
   Phe Val Leu Thr Ala Ala His Cys Leu Arg Asp Ile Pro Gln Arg Leu
   65                  70                  75                  80
   Val Asn Val Val Leu Gly Ala His Asn Val Arg Thr Gln Glu Pro Thr
                   85                  90                  95
   Gln Gln His Phe Ser Val Ala Gln Val Phe Leu Asn Asn Tyr Asp Ala
   100 105 110
   Glu Asn Lys Leu Asn Asp Val Leu Leu Ile Gln Leu Ser Ser Pro Ala
   115 120 125
   Asn Leu Ser Ala Ser Val Ala Thr Val Gln Leu Pro Gln Gln Asp Gln
   130 135 140
   Pro Val Pro His Gly Thr Gln Cys Leu Ala Met Gly Trp Gly Arg Val
   145 150 155 160
   Gly Ala His Asp Pro Pro Ala Gln Val Leu Gln Glu Leu Asn Val Thr
   165 170 175
   Val Val Thr Phe Phe Cys Arg Pro His Asn Ile Cys Thr Phe Val Pro
   180 185 190
   Arg Arg Lys Ala Gly Ile Cys Phe Gly Asp Ser Gly Gly Pro Leu Ile
   195 200 205
   Cys Asp Gly Ile Ile Gln Gly Ile Asp Ser Phe Val Ile Trp Gly Cys
   210 215 220
   Ala Thr Arg Leu Phe Pro Asp Phe Phe Thr Arg Val Ala Leu Tyr Val
   225 230 235 240
   Asp Trp Ile Arg Ser Thr Leu Arg Arg Val Glu Ala Lys Gly Arg Pro
   245 250 255
<210> 2
   <211> 379
   <212> PRT
   <213> Homo sapiens
<220>
   <221> MISC_FEATURE
   <223> Leukocyte elastase inhibitor
<400> 2
   Met Glu Gln Leu Ser Ser Ala Asn Thr Arg Phe Ala Leu Asp Leu Phe
   1 5 10 15
   Leu Ala Leu Ser Glu Asn Asn Pro Ala Gly Asn Ile Phe Ile Ser Pro
               20                  25                  30
   Phe Ser Ile Ser Ser Ala Met Ala Met Val Phe Leu Gly Thr Arg Gly
           35                  40                  45
   Asn Thr Ala Ala Gln Leu Ser Lys Thr Phe His Phe Asn Thr Val Glu
       50                  55                  60
   Glu Val His Ser Arg Phe Gln Ser Leu Asn Ala Asp Ile Asn Lys Arg
   65                  70                  75                  80
   Gly Ala Ser Tyr Ile Leu Lys Leu Ala Asn Arg Leu Tyr Gly Glu Lys
                   85                  90                  95
   Thr Tyr Asn Phe Leu Pro Glu Phe Leu Val Ser Thr Gln Lys Thr Tyr
               100                 105                 110
   Gly Ala Asp Leu Ala Ser Val Asp Phe Gln His Ala Ser Glu Asp Ala
           115                 120                 125
   Arg Lys Thr Ile Asn Gln Trp Val Lys Gly Gln Thr Glu Gly Lys Ile
       130                 135                 140
   Pro Glu Leu Leu Ala Ser Gly Met Val Asp Asn Met Thr Lys Leu Val
   145                 150                 155                 160
   Leu Val Asn Ala Ile Tyr Phe Lys Gly Asn Trp Lys Asp Lys Phe Met
                   165                 170                 175
   Lys Glu Ala Thr Thr Asn Ala Pro Phe Arg Leu Asn Lys Lys Asp Arg
               180                 185                 190
   Lys Thr Val Lys Met Met Tyr Gln Lys Lys Lys Phe Ala Tyr Gly Tyr
           195                 200                 205
   Ile Glu Asp Leu Lys Cys Arg Val Leu Glu Leu Pro Tyr Gln Gly Glu
       210                 215                 220
   Glu Leu Ser Met Val Ile Leu Leu Pro Asp Asp Ile Glu Asp Glu Ser
   225                 230                 235                 240
   Thr Gly Leu Lys Lys Ile Glu Glu Gln Leu Thr Leu Glu Lys Leu His
                   245                 250                 255
   Glu Trp Thr Lys Pro Glu Asn Leu Asp Phe Ile Glu Val Asn Val Ser
               260                 265                 270
   Leu Pro Arg Phe Lys Leu Glu Glu Ser Tyr Thr Leu Asn Ser Asp Leu
           275                 280                 285
   Ala Arg Leu Gly Val Gln Asp Leu Phe Asn Ser Ser Lys Ala Asp Leu
       290                 295                 300
   Ser Gly Met Ser Gly Ala Arg Asp Ile Phe Ile Ser Lys Ile Val His
   305                 310                 315                 320
   Lys Ser Phe Val Glu Val Asn Glu Glu Gly Thr Glu Ala Ala Ala Ala
                   325                 330                 335
   Thr Ala Gly Ile Ala Thr Phe Cys Met Leu Met Pro Glu Glu Asn Phe
               340                 345                 350
   Thr Ala Asp His Pro Phe Leu Phe Phe Ile Arg His Asn Ser Ser Gly
           355                 360                 365
   Ser Ile Leu Phe Leu Gly Arg Phe Ser Ser Pro
       370                 375

## Description

The present invention relates to the diagnosis of colorectal cancer. It discloses the use of the protein PRN3 (proteinase3) bound to the serpin-type proteinase inhibitor ILEU (Leukocyte elastase inhibitor) (= PRN3/ILEU) in the diagnosis of colorectal cancer. Furthermore, it especially relates to a method for diagnosis of colorectal cancer from a liquid sample, derived from an individual by measuring PRN3/ILEU in said sample. Measurement of PRN3/ILEU can, e.g., be used in the early detection or diagnosis of colorectal cancer.

Cancer remains a major public health challenge despite progress in detection and therapy. Amongst the various types of cancer, colorectal cancer (= CRC) is one of the most frequent cancers in the Western world.

The earlier cancer can be detected/diagnosed, the better is the overall survival rate. This is especially true for CRC. The prognosis in advanced stages of tumor is poor. More than one third of the patients will die from progressive disease within five years after diagnosis, corresponding to a survival rate of about 40% for five years. Current treatment is only curing a fraction of the patients and clearly has the best effect on those patients diagnosed in an early stage of disease.

With regard to CRC as a public health problem, it is essential that more effective screening and preventative measures for colorectal cancer be developed.

The earliest detection procedures available at present for colorectal cancer involve using tests for fecal blood or endoscopic procedures. However, significant tumor size must typically exist before fecal blood is detected. The sensitivity of the guaiac-based fecal occult blood tests is ∼26%, which means 74% of patients with malignant lesions will remain undetected (Ahlquist, D.A., Gastroenterol. Clin. North Am. 26 (1997) 41-55). The visualization of precancerous and cancerous lesions represents the best approach to early detection, but colonoscopy is invasive with significant costs, risks, and complications (Silvis, S.E., et al., JAMA 235 (1976) 928-930; Geenen, J.E., et al., Am. J. Dig. Dis. 20 (1975) 231-235; Anderson, W.F., et al., J. Natl. Cancer Institute 94 (2002) 1126-1133).

In the recent years a tremendous amount of so-called colon specific or even so-called colorectal cancer specific genes has been reported. The vast majority of the corresponding research papers or patent applications are based on data obtained by analysis of RNA expression patterns in colon (cancer) tissue versus a different tissue or an adjacent normal tissue, respectively. Such approaches may be summarized as differential mRNA display techniques.

As an example for data available from mRNA-display techniques, WO 01/96390 shall be mentioned and discussed. This application describes and claims more than two hundred isolated polynucleotides and the corresponding polypeptides as such, as well as their use in the detection of CRC. However, it is-general knowledge that differences on the level of mRNA are not mirrored by the level of the corresponding proteins. A protein encoded by a rare mRNA may be found in very high amounts and a protein encoded by an abundant mRNA may nonetheless be hard to detect and find at all. This lack of correlation between mRNA-level and protein level is due to reasons like mRNA stability, efficiency of translation, stability of the protein, etc.

There also are recent approaches investigating the differences in protein patterns between different tissues or between healthy and diseased tissue in order to identify candidate marker molecules which might be used in the diagnosis of CRC. Brünagel, G., et al., Cancer Research 62 (2002) 2437-2442 have identified seven nuclear matrix proteins which appear to be more abundant in CRC tissue as compared to adjacent normal tissue. No data from liquid samples obtained from an individual are reported.

WO 02/078636 reports about nine colorectal cancer-associated spots as found by surface-enhanced laser desorption and ionization (SELDI). These spots are seen more frequently in sera obtained from patients with CRC as compared to sera obtained from healthy controls. However, the identity of the molecule(s) comprised in such spot, e.g., its (their sequence), is not known.

Despite the large and ever growing list of candidate protein markers in the field of CRC, to date clinical/diagnostic utility of these molecules is not known. In order to be of clinical utility a new diagnostic marker as a single marker should be at least as good as the best single marker known in the art. Or, a new marker should lead to a progress in diagnostic sensitivity and/or specificity either if used alone or in combination with one or more other markers, respectively. The diagnostic sensitivity and/or specificity of a test is best assessed by its receiver-operating characteristics, which will be described in detail below.

At present, only diagnostic blood tests based on the detection of carcinoembryonic antigen (CEA), a tumor-associated glycoprotein, are available to assist diagnosis in the field of CRC. CEA is increased in 95% of tissue samples obtained from patients with colorectal, gastric, and pancreatic cancers and in the majority of breast, lung, and head and neck carcinomas (Goldenberg, D.M., et al., J. Natl. Cancer Inst. (Bethesda) 57 (1976) 11-22). Elevated CEA levels have also been reported in patients with nonmalignant disease, and many patients with colorectal cancer have normal CEA levels in the serum, especially during the early stage of the disease (Carriquiry, L.A., and Pineyro, A., Dis. Colon Rectum 42 (1999) 921-929; Herrera, M.A., et al., Ann. Surg. 183 (1976) 5-9; Wanebo, H.J., et al., N. Engl. J. Med. 299 (1978) 448-451). The utility of CEA as measured from serum or plasma in detecting recurrences is reportedly controversial and has yet to be widely applied (Martell, RE., et al., Int. J. Biol. Markers 13 (1998) 145-149; Moertel, C.G., et al., JAMA 270 (1993) 943-947).

In light of the available data, serum CEA determination possesses neither sensitivity nor the specificity to enable its use as a screening test for colorectal cancer in the asymptomatic population (Reynoso, G., et al., JAMA 220 (1972) 361-365; Sturgeon, C., Clinical Chemistry 48 (2002) 1151-1159).

Whole blood, serum or plasma are the most widely used sources of sample in clinical routine. The identification of an early CRC tumor marker that would allow reliable cancer detection or provide early prognostic information could lead to a diagnostic assay that would greatly aid in the diagnosis and in the management of this disease. Therefore, an urgent clinical need exists to improve the diagnosis of CRC from blood. It is especially important to improve the early diagnosis of CRC, since for patients diagnosed early on chances of survival are much higher as compared to those diagnosed at a progressed stage of disease.

It was the task of the present invention to investigate whether a new marker can be identified which may aid in CRC diagnosis.

Surprisingly, it has been found that use of PRN3 bound to the serpin-type proteinase inhibitor ILEU (PRN3/ILEU) can at least partially overcome the problems known from the state of the art.

The present invention therefore relates to a method for the diagnosis of colorectal cancer comprising the steps of a) providing a liquid sample obtained from an individual, b) contacting said sample with a first specific binding agent for proteinase 3 (PRN3) and a second specific binding agent for the serpin-type proteinase inhibitor leukocyte elastase inhibitor (ILEU) under conditions appropriate for formation of a complex between said first and second binding agent and PRN3 bound to ILEU (PRN3/ILEU), and c) correlating the amount of complex formed in (b) to the diagnosis of colorectal cancer.

Another preferred embodiment of the invention is a method for the diagnosis of colorectal cancer comprising the steps of a) contacting a liquid sample obtained from an individual with a first specific binding agent for proteinase 3 (PRN3) and a second specific binding agent for the serpin-type proteinase inhibitor leukocyte elastase inhibitor (ILEU) under conditions appropriate for formation of a complex between said first and second binding agent and PRN3 bound to ILEU (PRN3/ILEU), and b) correlating the amount of complex formed in (a) to the diagnosis of colorectal cancer.

As the skilled artisan will appreciate, any such diagnosis is made in vitro. The patient sample is discarded afterwards. The patient sample is solely used for the in vitro diagnostic method of the invention and the material of the patient sample is not transferred back into the patient's body. Typically, the sample is a liquid sample.

The protein PRN3 (proteinase 3, also known as myeloblastin precursor or Wegener's autoantigen; Swiss-PROT: P24158) is characterized by the sequence given SEQ ID NO:1. Proteinase 3 is a serine proteinase found in azurophilic granules of polymorphonuclear neutrophils (Baggiolini, M., et al., Agents Actions 8 (1978) 3-10). Proteinase 3 has been characterized and named independently as p29b (Campanelli, D., et al., J. Clin. Invest. 85 (1990) 904-915) and myeloblastin (Bories, D., et al., Cell 59 (1989) 959-968), which were later found to be identical to proteinase 3. PRN3 is a protein of about 29 kDa and exists as multiple Mr species, probably due to differences in glycosylation (Wilde, C.G., et al., J. Biol. Chem. 265 (1990) 2038-2041).

PRN3 has been associated with a number of human diseases. It is the target antigen of the cytoplasmic pattern of antineutrophil cytoplasmic autoantibodies (c-ANCA) detected in the circulation of Wegener's granulomatosis (WG) patients (Ludemann, J., et al., J. Exp. Med. 171 (1990) 357-362). PRN3 also is involved in growth and differentiation of leukemia cells (Bories, D., et al., Cell 59 (1989) 959-968), and animal models have indicated a role for PRN3 in emphysema (Kao, R.C., et al., J. Clin. Invest. 82 (1988) 1963-1973).

PRN3 can be detected on the cell surface following activation by tumor necrosis factor alpha (TNFα) in vitro (Charles, L.A., et al., J. Leuk. Biol. 50 (1991) 539-546), and PRN3 has been reported to release TNFα from cell surfaces in vitro (Robache-Gallea et al., 1995). PRN3 is also found on the surface of cells in patients with ANCA-associated vasculitides (Csernok, E., et al., Advan. Exp. Med. Biol. 336 (1993) 45-50)

The sequence of ILEU (Leukocyte elastase inhibitor, also known as monocyte/neutrophil elastase inhibitor; Swiss-PROT P30740) is given in SEQ ID NO:2. It is a 42 kDa protein of the serpin superfamily (Remold-O'Donnell, E., et al., PNAS 89 (1992) 5635-5639). Serpin-type inhibitors are described to bind covalently to their target proteinases and form stable serpin-proteinase complexes, which are not dissociated when treated with SDS (Olson, S., et al., JBC 270 (1995) 30007-30017).

In particular, ILEU was shown to inhibit, amongst others, PRN3 (proteinase-3) fast and efficiently (Cooley, J., et al., Biochem. 40 (2001) 15762-15770). This inhibition is accomplished through a covalent binding in a 1:1 stoichiometry. SDS-PAGE revealed that the ILEU-PRN3-complex formed was resistant to SDS treatment (Cooley, J., et al., Biochem. 40 (2001) 15762-15770).

As obvious to the skilled artisan, the present invention shall not be construed to be limited to the full-length protein PRN3 of SEQ ID NO: 1 bound to the full-length protein ILEU of SEQ NO ID:2. ILEU-PRN3-complexes also may comprise physiological or artificial fragments of PRN3 or ILEU, secondary modifications of PRN3 or ILEU, as well as allelic variants of PRN3 or ILEU. Therefore, PRN3 as well as fragments, modifications and variants of PRN3 being bound to ILEU or to fragments, modifications and variants thereof are also encompassed by the present invention.

Artificial fragments of PRN3 preferably encompass a peptide produced synthetically or by recombinant techniques, which at least comprises one epitope of diagnostic interest consisting of at least 6 contiguous amino acids as derived from the sequence disclosed in SEQ ID NO:1. Such fragment may advantageously be used for generation of antibodies or as a standard in an immunoassay. More preferred the artificial fragment comprises at least two epitopes of interest appropriate for setting up a sandwich immunoassay. Similarly, the present invention encompasses antibodies generated using a peptide as above for PRN3 but derived from the sequence disclosed in SEQ ID NO:2, whereby the peptide represents at least one ILEU epitope.

A preferred embodiment of the present invention is a method for the diagnosis of colorectal cancer comprising the steps of a) providing a liquid sample obtained from an individual, b) contacting said sample with a first specific binding agent for proteinase 3 (PRN3) and a second specific binding agent for the serpin-type proteinase inhibitor leukocyte elastase inhibitor (ILEU) under conditions appropriate for formation of a complex between said first and second binding agent and PRN3 bound to ILEU (PRN3/ILEU), and c) correlating the amount of complex formed in (b) to the diagnosis of colorectal cancer. Even more preferred that method is a sandwich immunoassay using either a PRN3-specific antibody for capturing and an ILEU-specific antibody for detecting PRN3/ILEU, or an ILEU-specific antibodiey for capturing and a PRN3-specific antibody for detecting PRN3/ILEU.

It is anticipated that not only PRN3/ILEU can serve as a marker for CRC but also free PRN3, alone or in combination with PRN3/ILEU. For this reason, the findings on which this invention is based in total provides for three different specific targets: (i) PRN3/ILEU alone, (ii) PRN3/ILEU and free PRN3, and (iii) unbound PRN3 alone. The three targets can be used individually as markers or a ratio of the amounts of at least two of the targets (i), (ii) and (iii) can be determined. The ratio can serve as a parameter characterizing CRC.

Further disclosed is a method for the diagnosis of colorectal cancer comprising the steps of (a) providing a liquid sample obtained from an individual, b) contacting said sample with a binding agent against an epitope shared by PRN3 and PRN3/ILEU under conditions appropriate for formation of a complex between said binding agent and PRN3 as well as PRN3/ILEU, and (c) correlating the amount of complex formed in (b) to the diagnosis of colorectal cancer. This way all PRN3 present in the liquid sample ("total PRN3") is measured.

Further disclosed is a method for the diagnosis of colorectal cancer comprising the steps of (a) providing a liquid sample obtained from an individual, b) contacting said sample with a binding agent against an epitope present on unbound PRN3 but not on PRN3/ILEU under conditions appropriate for formation of a complex between said binding agent and PRN3, and (c) correlating the amount of complex formed in (b) to the diagnosis of colorectal cancer. As the skilled artisan will appreciate at least one binding reagent is required which recognizes an epitope on PRN3 absent, e.g., masked if PRN3 is bound to ILEU. In a sandwich assay at least one such reagent is either used as capture or as detection reagent

Any of the novel markers PRN3/ILEU, total PRN3 and/or unbound PRN3 alone or in combination may be used for monitoring as well as for screening purposes.

When used in patient monitoring the diagnostic method according to the present invention may help to assess tumor load, efficacy of treatment and tumor recurrence in the follow-up of patients. Increased levels of PRN3/ILEU are directly correlated to tumor burden. After chemotherapy a short term (few hours to 14 days) increase in PRN3/ILEU may serve as an indicator of tumor cell death. In the follow-up of patients (from 3 months to 10 years) an increase of PRN3/ILEU can be used as an indicator for tumor recurrence.

In a preferred embodiment the diagnostic method according to the present invention is used for screening purposes. I.e., it is used to assess subjects without a prior diagnosis of CRC by measuring the level of PRN3/ILEU and correlating the level measured to the presence or absence of CRC.

Colorectal cancer most frequently progresses from adenomas (polyps) to malignant carcinomas. The different stages of CRC used to be classified according to Dukes' stages A to D.

The staging of cancer is the classification of the disease in terms of extent, progression, and severity. It groups cancer patients so that generalizations can be made about prognosis and the choice of therapy.

Today, the TNM system is the most widely used classification of the anatomical extent of cancer. It represents an internationally accepted, uniform staging system. There are three basic variables: T (the extent of the primary tumor), N (the status of regional lymph nodes) and M (the presence or absence of distant metastases). The TNM criteria are published by the UICC (International Union Against Cancer), Sobin, L.H., Wittekind, Ch. (eds): TNM Classification of Malignant Tumours, fifth edition, 1997.

What is especially important is, that early diagnosis of CRC translates to a much better prognosis. Malignant tumors of the colorectum arise from benign tumors, i.e. from adenoma. Therefore, best prognosis have those patients diagnosed at the adenoma stage. Patients diagnosed as early as in stage Tᵢₛ, N0, M0 or T1-3; N0; M0, if treated properly have a more than 90% chance of survival 5 years after diagnosis as compared to a 5-years survival rate of only 10% for patients diagnosed when distant metastases are already present

In the sense of the present invention early diagnosis of CRC refers to a diagnosis at a pre-malignant state (adenoma) or at a tumor stage where no metastases at all (neither proximal nor distal), i.e., adenoma, Tᵢₛ, N0 M0 or T1-4; N0; M0 are present. Tᵢₛ denotes carcinoma *in situ.*

In a preferred embodiment the detection of PRN3/ILEU is used to diagnose CRC as early as in the adenoma stage.

It is further preferred, that CRC is diagnosed when it has not yet fully grown through the bowel wall and thus neither the visceral peritoneum is perforated nor other organs or structures are invaded, i.e., that diagnosis is made at stage Tᵢₛ, N0, M0 or T1-3; N0; M0(=Tᵢₛ-3; N0; M0).

The diagnostic method according to the present invention is based on a liquid sample which is derived from an individual. Unlike to methods known from the art PRN3/ILEU is specifically measured from this liquid sample by use of a specific binding agent

A specific binding agent is, e.g., a receptor for PRN3, ILEU or PRN3/ILEU, a lectin binding to PRN3, ILEU or PRN3/ILEU or an antibody to PRN3, ILEU or PRN3/ILEU. A specific binding agent has at least an affinity of 10⁷ l/mol for its corresponding target molecule. The specific binding agent preferably has an affinity of 10⁸ l/mol or even more preferred of 10⁹ l/mol for its target molecule. As the skilled artisan will appreciate the term specific is used to indicate that other biomolecules present in the sample do not significantly bind to with the binding agent specific for PRN3, ILEU or PRN3/ILEU. Preferably, the level of binding to a biomolecule other than the target molecule results in a binding affinity which is only 10%, more preferably only 5% of the affinity of the target molecule or less. A most preferred specific binding agent will fulfill both the above minimum criteria for affinity as well as for specificity.

A pair of specific binding agents preferably comprises a first antibody reactive with PRN3 and a second antibody reactive with ILEU such that the pair of antibodies is capable of forming a complex with PRN3/ILEU. Furthermore, a specific binding agent preferably is an antibody specifically reactive with PRN3/ILEU but not PRN3 or ILEU alone. Also encompassed by the present invention is a specific binding agent directed against unbound PRN3, whereby the specific binding agent preferably is an antibody reactive with an epitope of PRN3 which is masked when PRN3 is bound to ILEU.

The term antibody refers to a polyclonal antibody, a monoclonal antibody, fragments of such antibodies, as well as genetic constructs comprising the binding domain of an antibody. Any antibody fragment retaining the above criteria of a specific binding agent can be used. Antibodies are generated by state of the art procedures, e.g., as described in Tijssen (Tijssen, P., Practice and theory of enzyme immunoassays 11 (1990) the whole book, especially pages 43-78; Elsevier, Amsterdam). In addition, the skilled artisan is well aware of methods based on immunosorbents that can be used for the specific isolation of antibodies. By these means the quality of polyclonal antibodies and hence their performance in immunoassays can be enhanced. (Tijssen, P., *supra*, pages 108-115).

For the achievements as disclosed in the present invention polyclonal antibodies raised in rabbits have been used. However, clearly also polyclonal antibodies from different species, e.g. rats or guinea pigs, as well as monoclonal antibodies can also be used. Since monoclonal antibodies can be produced in any amount required with constant properties, they represent ideal tools in development of an assay for clinical routine. The generation and use of monoclonal antibodies to PRN3, ILEU and PRN3/ILEU in a method according to the present invention is yet another preferred embodiment.

As the skilled artisan will appreciate now, that PRN3/ILEU has been identified as a marker which is useful in the diagnosis of CRC, alternative ways may be used to reach a result comparable to the achievements of the present invention. For example, alternative strategies to generate antibodies may be used. Such strategies comprise amongst others the use of synthetic peptides, representing an epitope of PRN3, ILEU or PRN3/ILEU for immunization. Alternatively, DNA Immunization also known as DNA vaccination may be used.

For measurement the liquid sample obtained from an individual is incubated with the specific binding agent or pair of binding agents for PRN3/ILEU under conditions appropriate for formation of a complex of the binding agent or pair of binding agents and PRN3/ILEU. Such conditions need not be specified, since the skilled artisan without any inventive effort can easily identify such appropriate incubation conditions.

As a final step according to the method disclosed in the present invention the amount of complex is measured and correlated to the diagnosis of CRC. As the skilled artisan will appreciate there are numerous methods to measure the amount of formed complex consisting of the specific binding agent or pair of specific binding agents and PRN3/ILEU, all described in detail in relevant textbooks (cf., e.g., Tijssen P., *supra*, or Diamandis, et al., eds. (1996) Immunoassay, Academic Press, Boston).

Preferably PRN3/ILEU is detected in a sandwich type assay format. In such assay a first specific binding agent is used to capture PRN3/ILEU on the one side and a second specific binding agent, which is labeled to be directly or indirectly detectable is used on the other side.

As mentioned above, it has surprisingly been found that PRN3/ILEU can be measured from a liquid sample obtained from an individual sample. No tissue and no biopsy sample is required to apply the marker PRN3/ILEU in the diagnosis of CRC.

In a preferred embodiment the method according to the present invention is practiced with serum as liquid sample material.

In a further preferred embodiment the method according to the present invention is practiced with plasma as liquid sample material.

In a further preferred embodiment the method according to the present invention is practiced with whole blood as liquid sample material.

Furthermore stool can be prepared in various ways known to the skilled artisan to result in a liquid sample as well. Such sample liquid derived from stool also represents a preferred embodiment according to the present invention.

Whereas application of routine proteomics methods to tissue samples, leads to the identification of many potential marker candidates for the tissue selected, the inventors of the present invention have surprisingly been able to detect PRN3/ILEU in a bodily fluid sample. Even more surprising they have been able to demonstrate that the presence of PRN3/ILEU in such liquid sample obtained from an individual can be correlated to the diagnosis of colorectal cancer.

Antibodies to PRN3/ILEU with great advantage can be used in established procedures, e.g., to detect colorectal cancer cells in situ, in biopsies, or in immunohistological procedures.

Preferably, an antibody to PRN3/ILEU is used in a qualitative (PRN3/ILEU present or absent) or quantitative (PRN3/ILEU amount is determined) immunoassay.

Based on quantitative determination of PRN3/ILEU and total PRN3 or unbound PRN3 the ratio between these markers can be correlated to the diagnosis of colorectal cancer.

Measuring the level of protein PRN3/ILEU has proven very advantageous in the field of CRC. Therefore, in a further preferred embodiment, the present invention relates to use of PRN3/ILEU as a marker molecule in the diagnosis of colorectal cancer from a liquid sample obtained from an individual.

The term marker molecule is used to indicate that an increased level of the analyte PRN3/ILEU as measured from a bodily fluid of an individual marks the presence of CRC.

It is especially preferred to use the novel marker PRN3/ILEU in the early diagnosis of colorectal cancer.

The use of PRN3/ILEU itself, represents a significant progress to the challenging field of CRC diagnosis. Combining measurements of PRN3/ILEU with other known markers, like CEA, or with other markers of CRC yet to be discovered, leads to further improvements. Therefore in a further preferred embodiment the present invention relates to the use of PRN3/ILEU as a marker molecule for colorectal cancer in combination with one or more marker molecules for colorectal cancer in the diagnosis of colorectal cancer from a liquid sample obtained from an individuaL In this regard, the expression "one or more" denotes 1 to 10, preferably 1 to 5, more preferred 3. Preferred selected other CRC markers with which the measurement of PRN3/ILEU may be combined are CEA, CA 19-9, CA 72-4, and/or CA 242. Thus, a very much preferred embodiment of the present invention is the use of protein PRN3 or PRN3/ELEU as a marker molecule for colorectal cancer in combination with one or more marker molecules for colorectal cancer in the diagnosis of colorectal cancer from a liquid sample obtained from an individual, whereby the at least one other marker molecule is selected from the group consisting of CEA, CA 19-9, CA 72-4, and CA 242.

Diagnostic reagents in the field of specific binding assays, like immunoassays, usually are best provided in the form of a kit, which comprises the specific binding agent and the auxiliary reagents required to perform the assay.

Accuracy of a test is best described by its receiver-operating characteristics (ROC) (see especially Zweig, M. H., and Campbell, G., Clin. Chem. 39 (1993) 561-577). The ROC graph is a plot of all of the sensitivity/specificity pairs resulting from continuously varying the decision thresh-hold over the entire range of data observed.

The clinical performance of a laboratory test depends on its diagnostic accuracy, or the ability to correctly classify subjects into clinically relevant subgroups. Diagnostic accuracy measures the test's ability to correctly distinguish two different conditions of the subjects investigated. Such conditions are for example health and disease or benign versus malignant disease.

In each case, the ROC plot depicts the overlap between the two distributions by plotting the sensitivity versus 1 - specificity for the complete range of decision thresholds. On the y-axis is sensitivity, or the true-positive fraction [defined as (number of true-positive test results) (number of true-positive + number of false-negative test results)]. This has also been referred to as positivity in the presence of a disease or condition. It is calculated solely from the affected subgroup. On the x-axis is the false-positive fraction, or 1 - specificity [defined as (number of false-positive results) / (number of true-negative + number of false-positive results)]. It is an index of specificity and is calculated entirely from the unaffected subgroup. Because the true- and false-positive fractions are calculated entirely separately, by using the test results from two different subgroups, the ROC plot is independent of the prevalence of disease in the sample. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. (If the ROC plot falls completely below the 45° diagonal, this is easily remedied by reversing the criterion for "positivity" from "greater than" to "less than" or vice versa.) Qualitatively, the closer the plot is to the upper left corner, the higher the overall accuracy of the test

One convenient goal to quantify the diagnostic accuracy of a laboratory test is to express its performance by a single number. The most common global measure is the area under the ROC plot. By convention, this area is always ≥ 0.5 (if it is not, one can reverse the decision rule to make it so). Values range between 1.0 (perfect separation of the test values of the two groups) and 0.5 (no apparent distributional difference between the two groups of test values). The area does not depend only on a particular portion of the plot such as the point closest to the diagonal or the sensitivity at 90% specificity, but on the entire plot. This is a quantitative, descriptive expression of how close the ROC plot is to the perfect one (area = 1.0).

Clinical utility of the novel marker PRN3/ILEU has been assessed in comparison to and in combination with the established marker CEA using a receiver operator curve analysis (ROC; Zweig, M. H., and Campbell, G., Clin. Chem. 39 (1993) 561-577). This analysis has been based on well-defined patient cohorts consisting of 50 samples each from patients in T1-3; N0; M0, more progressed tumor, i.e., T4 and/or various severity of metastasis (N+ and/or M+), and healthy controls, respectively.

The diagnostic method based on measurement of PRN3/ILEU alone in comparison to the established marker CEA alone has been found to have an at least as good a diagnostic accuracy (sensitivity/specificity profile) as demonstrated by the area under the curve.

### Description of the Figures

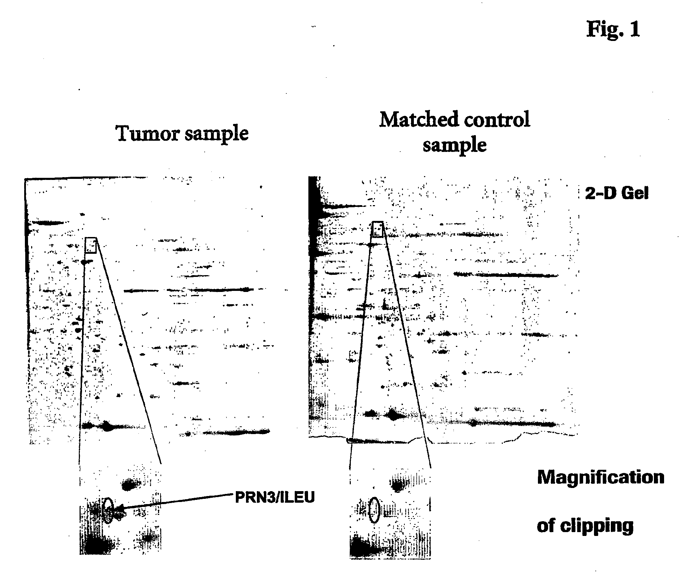

- **Figure 1**: Identification of intact PRN3/ILEU with an apparent MW of 54 kDa in colon tumor tissue. Figure 1 shows a typical example of a 2D-gel, loaded with a tumor sample (left side), and a gel, loaded with a matched control sample (right side) obtained from adjacent healthy mucosa. The circle in the enlarged section of these gels indicates the position for PRN3/ILEU. The PRN3/ILEU complex was not detectable by the same method in healthy mucosa.

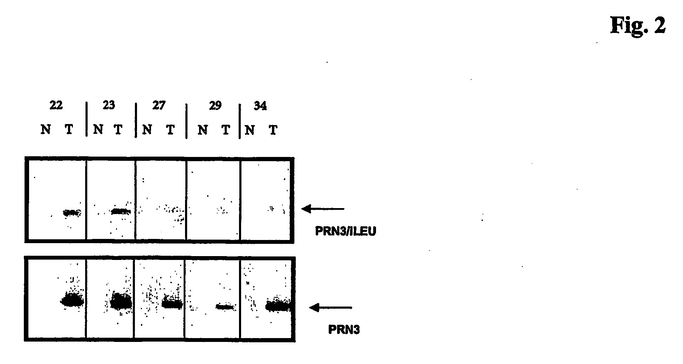

- **Figure 2**: Figure 2 shows a typical example of a Western-Blot. The gel was loaded with tissue lysates from colorectal tumor tissue and adjacent healthy control tissue from 5 patients (subject 22: colon ca, Dukes B; subject 23: colon ca, Dukes B; subject 27: rectum ca, Dukes B; subject 29: rectum ca, Dukes A, and subject 34: colon ca, Dukes B). Presence of the PRN3-ILEU complex (upper clipping) or of uncomplexed PRN3 (lower clipping) in the samples was tested using a polyclonal rabbit anti-ILEU (upper clipping) or polyclonal anti-PRN3 (lower clipping) serum. Lanes containing tumor lysates are indicated with "T", lanes containing normal control tissue with "N". The arrows indicate the position in the gels of the PRN3-ILEU (apparent MW of -70 kDa) and the uncomplexed PRN3 (apparent MW of -28 kDa) band. All tumor samples gave a strong signal at the positions of PRN3-ILEU and PRN3, whereas only a weak or no signal could be detected in the lysates from adjacent normal control tissue.
The apparent molecular weight of the PRN3-ILEU complex in this experiment was about 70 kDa and therefore different to the apparent molecular weight of the PRN3-ILEU complex as determined on the 2D-gels where an apparent molecular weight of about 54 kDa was determined (see Figure 1). The determination of the apparent molecular weight in the Western-blot experiment is regarded as more precise since in this experiment a molecular weight marker is included. The 2D-gels, in contrast, do not contain a molecular weight standard and therefore estimations of the molecular weight of a given spot are clearly less precise. The molecular weight of the PRN3- ILEU complex as determined in the Western-Blot experiment is well in accordance with the theoretical molecular weight.

### Abbreviations

- ABTS: 2,2'-Azino-di- [3-ethylbenzthiazoline sulfonate (6)] diammonium salt
- BSA: bovine serum albumin
- cDNA: complementary DNA
- CHAPS: (3-[(3-Cholamidopropyl)-dimethylammonio]- 1-propane-sulfonate)
- DMSO: dimethyl sulfoxide
- DTT: dithiothreitol
- EDTA: ethylene diamine tetraacetic acid
- ELISA: enzyme-linked immunosorbent assay
- HRP: horseradish peroxidase
- IAA: iodoacetamid
- IgG: immunoglobulin G
- IEF: isoelectric focussing
- IPG: immobilized pH gradient
- LDS: lithium dodecyl sulfate
- MALDI-TOF: matrix-assisted laser desorption/ionisation-time of flight mass spectrometry
- MES: mesityl, 2,4,6-trimethylphenyl
- OD: optical density
- PAGE: polyacrylamide gel electrophoresis
- PBS: phosphate buffered saline
- PI: isoelectric point
- RTS: rapid translation system
- SDS: sodium dodecyl sulfate

### Example 1

### Identification of PRN3/ILEU as a potential colorectal cancer marker

### Sources of tissue

In order to identify tumor-specific proteins as potential diagnostic markers for colorectal cancer, analysis of three different kinds of tissue using proteomics methods is performed.

In total, tissue specimen from 10 patients suffering from colorectal cancer are analyzed. From each patient three different tissue types are collected from therapeutic resections: tumor tissue (> 80% tumor) (T), adjacent healthy tissue (N) and stripped mucosa from adjacent healthy mucosa (M). The latter two tissue types serve as matched healthy control samples. Tissues are immediately snap frozen after resection and stored at - 80°C before processing. Tumors are diagnosed by histopathological criteria.

### Tissue preparation

0.8-1.2 g of frozen tissue are put into a mortar and completely frozen by liquid nitrogen. The tissue is pulverized in the mortar, dissolved in the 10-fold volume (w/v) of lysis buffer (40 mM Na-dtrate, 5 mM MgCl₂, 1% Genapol X-080, 0.02% Na-azide, Complete^{®} EDTA-free [Roche Diagnostics GmbH, Mannheim, Germany, Cat No.1873 580]) and subsequently homogenized in a Wheaton^{®} glass homogenizer (20 x loose fitting, 20 x tight fitting). 3 ml of the homogenate are subjected to a sucrose-density centrifugation (10-60% sucrose) for 1 h at 4500 x g. After this centrifugation step three fractions are obtained. The fraction on top of the gradient contains the soluble proteins and is used for further analysis.

### Isoelectric focussing (IEF) and SDS-PAGE

For IEF, 3 ml of the suspension are mixed with 12 ml sample buffer (7 M urea, 2 M thiourea, 2% CHAPS, 0.4% IPG buffer pH 6-9, 0:5% DTT) and incubated for 1 h. The samples are concentrated in an Amicon^{®} Ultra-15 device (Millipore GmbH, Schwalbach, Germany) and the protein concentration is determined using the Bio-Rad^{®} protein assay (Cat.No. 500-0006; Bio-Rad Laboratories GmbH, München, Germany) following the instructions of the supplier's manual. To a volume corresponding to 1.5 mg of protein sample buffer is added to a final volume of 350 µl. This solution is used to rehydrate IPG strips pH6-9 (Amersham Biosciences, Freiburg, Germany) overnight. The IEF is performed using the following gradient protocol: 1.) 1 minute to 500 V; 2.) 2 h to 3,500 V; 3.) 22 h at constant 3,500 V giving rise to 82 kVh. After IEF, strips are stored at -80°C or directly used for SDS-PAGE.

Prior to SDS-PAGE the strips are incubated in equilibration buffer (6M urea, 50 mM Tris/HCl, pH 8.8, 30% glycerol, 2% SDS), for reduction DDT (15 min, + 50 mg DTT/10 ml), and for alkylation IAA (15 min, + 235 mg iodacetamide/10 ml) is added. The strips are put on 12.5% polyacrylamide gels and subjected to electrophoresis at 1 W/gel for 1 h and thereafter at 17 W/gel. Subsequently, the gels are fixed (50% methanol, 10% acetate) and stained overnight with Novex^{™} Colloidal Blue Staining Kit (Invitrogen, Karlsruhe, Germany, Cat. No. LC6025, 45-7101).

### Detection of PRN3/ILEU as a potential marker for colorectal cancer

Each patient is analyzed separately by image analysis with the ProteomeWeaver^{®} software (Definiens AG, Germany, München). In addition, all spots of the gel are excised by a picking robot and the proteins present in the spots are identified by MALDI-TOF mass spectrometry (Ultraflex^{™} Tof/Tof, Bruker Daltonik GmbH, Bremen, Germany). For each patient, 4 gels from the tumor sample are compared with 4 gels each from adjacent normal and stripped mucosa tissue and analyzed for distinctive spots corresponding to differentially expressed proteins. By this means, protein PRN3/ILEU is found to be specifically expressed or strongly overexpressed in tumor tissue and not detectable or less strongly expressed in healthy control tissue. It therefore - amongst many other proteins - qualifies as a candidate marker for use in the diagnosis of colorectal cancer.

### Example 2

### Generation of antibodies to PRN3 and ILEU

Polyclonal antibody to the colorectal cancer marker protein PRN3/ILEU is generated for further use of the antibody in the measurement of serum, and plasma, and blood and stool levels of PRN3/ILEU, total PRN3 and/or unbound PRN3 by immunodetection assays, e.g. Western Blotting and ELISA.

### Recombinant protein expression in E. coli

In order to generate antibodies to PRN3/ILEU, recombinant expression of each protein is performed for obtaining immunogens. Expression is done applying a combination of the RTS 100 expression system and E.coli. In a first step, the DNA sequences encoding PRN3 and ILEU are analyzed and recommendations for high yield cDNA silent mutational variants and respective PCR-primer sequences are obtained using the "ProteoExpert RTS E.coli HY" system. This is a commercial web based service (www.proteoexpert.com). Using the recommended primer pairs, the "RTS 100 E. coli Linear Template Generation Set, His-tag" (Roche Diagnostics GmbH, Mannheim, Germany, Cat.No. 3186237) system to generate linear PCR templates from the cDNA and for in-vitro transcription and expression of the nucleotide sequence coding for the PRN3 and ILEU proteins is used. For Western-blot detection and later purification, the expressed proteins contains a His-tag. The best expressing variants are identified. All steps from PCR to expression and detection are carried out according to the instructions of the manufacturer. The respective PCR products, containing all necessary T7 regulatory regions (promoter, ribosomal binding site and T7 terminator) are cloned into the pBAD TOPO^{®} vector (Invitrogen, Karlsruhe, Germany, Cat. No. K 4300/01) following the manufacturer's instructions. For expression using the T7 regulatory sequences, the construct is transformed into E. coli BL 21 (DE 3) (Studier, F.W., et aL, Methods Enzymol. 185 (1990) 60-89) and the transformed bacteria are cultivated in a 11 batch for protein expression. PRN3 and ILEU are expressed separately.

Purification of His-PRN3 and His-ILEU fusion proteins is done following standard procedures on a Ni-chelate column. Briefly, 11 of bacteria culture containing the expression vector for the His- His-PRN3 or the His-ILEU fusion protein is pelleted by centrifugation. The cell pellet is resuspended in lysis buffer, containing phosphate, pH 8.0, 7 M guanidium chloride, imidazole and thioglycerole, followed by homogenization using an Ultra-Turrax^{®}. Insoluble material is pelleted by high speed centrifugation and the supernatant is applied to a Ni-chelate chromatographic column. The column is washed with several bed volumes of lysis buffer followed by washes with buffer, containing phosphate, pH 8.0 and Urea.

Finally, bound antigen is eluted using a phosphate buffer containing SDS under acid conditions.

### Production of monoclonal antibodies against PRN3 and ILEU

### a) Immunization of mice

Two sets of 12 week old A/J mice are initially immunized intraperitoneally, one set with with 100 µg PRN3, the other with 100 µg ILEU. This is followed after 6 weeks by two further intraperitoneal immunizations at monthly intervals. In this process each mouse is administered 100 µg PRN3 or ILEU adsorbed to aluminum hydroxide and 10⁹ germs of *Bordetella pertussis*. Subsequently the last two immunizations are carried out intravenously on the 3rd and 2nd day before fusion using 100 µg PRN3 or ILEU, respectively, in PBS buffer for each.

### b) Fusion and cloning

Spleen cells of the mice immunized according to a) are fused with myeloma cells according to Galfre, G., and Milstein, C., Methods in Enzymology 73 (1981) 3-46. In this process ca. 1*10⁸ spleen cells of the immunized mouse are mixed with 2×10⁷ myeloma cells (P3X63-Ag8-653, ATCC CRL1580) and centrifuged (10 min at 300 x g and 4°C.). The cells are then washed once with RPMI 1640 medium without fetal calf serum (FCS) and centrifuged again at 400 g in a 50 ml conical tube. The supernatant is discarded, the cell sediment is gently loosened by tapping, 1 ml PEG (molecular weight 4000, Merck, Darmstadt) is added and mixed by pipetting. After 1 min in a water-bath at 37°C., 5 ml RPMI 1640 without FCS is added drop-wise at room temperature within a period of 4-5 min. Afterwards 5 ml RPMI 1640 containing 10% FCS is added drop-wise within ca. 1 min, mixed thoroughly, filled to 50 ml with medium (RPMI 1640+10% FCS) and subsequently centrifuged for 10 min at 400 g and 4°C. The sedimented cells are taken up in RPMI 1640 medium containing 10% FCS and sown in hypoxanthine-azaserine selection medium (100 mmol/l hypoxanthine, 1 µg/ml azaserine in RPMI 1640+10% FCS). Interleukin 6 at 100 U/ml is added to the medium as a growth factor.

After ca. 10 days the primary cultures are tested for specific antibody. Monoclonal antibody specific for unbound PRN3 is identified as being reactive with PRN3 but not reactive with PRN3/ILEU. PRN3- and ILEU-positive primary cultures are cloned in 96-well cell culture plates by means of a fluorescence activated cell sorter. In this process again interleukin 6 at 100 U/ml is added to the medium as a growth additive.

### c) Immunoglobulin isolation from the cell culture supernatants

The hybridoma cells obtained are sown at a density of 1×10⁵ cells per ml in RPMI 1640 medium containing 10% FCS and proliferated for 7 days in a fermenter (Thermodux Co., Wertheim/Main, Model MCS-104XL, Order No. 144-050). On average concentrations of 100 µg monoclonal antibody per ml are obtained in the culture supernatant. Purification of this antibody from the culture supernatant is carried out by conventional methods in protein chemistry (e.g. according to Bruck, C., et al., Methods in Enzymology 121 (1986) 587-695).

### Generation of polyclonal antibodies

### a) Immunization

Two sets of rabbits are immunized, one with PRN3 and one with ILEU. For immunization, a fresh emulsion of the protein solution (100 µg/ml PRN3 or ILEU, respectively) and complete Freund's adjuvant at the ratio of 1:1 is prepared. Each rabbit is immunized with 1 ml of the emulsion at days 1, 7, 14 and 30, 60 and 90. Blood is drawn and resulting anti-PRN3 and anti-ILEU serum used for further experiments as described in Examples 3 to 7.

### b) Purification of IgG (immunoglobulin G) from rabbit serum by sequential precipitation with caprylic acid and ammonium sulfate

One volume of rabbit serum is diluted with 4 volumes of acetate buffer (60 mM, pH 4.0). The pH is adjusted to 4.5 with 2 M Tris-base. Caprylic acid -(25 µl/ml of diluted sample) is added drop-wise under vigorous stirring. After 30 min the sample is centrifuged (13,000 x g, 30 min, 4°C), the pellet discarded and the supernatant collected. The pH of the supernatant is adjusted to 7.5 by the addition of 2 M Tris-base and filtered (0.2 µm).

The immunoglobulin in the supernatant is precipitated under vigorous stirring by the drop-wise addition of a 4 M ammonium sulfate solution to a final concentration of 2 M. The precipitated immunoglobulins are collected by centrifugation (8,000 x g,15 min, 4°C).

The supernatant is discarded. The pellet is dissolved in 10 mM NaH₂PO₄/NaOH, pH 7.5, 30 mM NaCl and exhaustively dialyzed. The dialysate is centrifuged (13,000 x g,15 min, 4°C) and filtered (0.2 µm).

### Biotinylation of polyclonal rabbit IgG

Polyclonal rabbit IgG is brought to 10 mg/ml in 10 mM NaH₂PO₄/NaOH, pH 7.5, 30 mM NaCl. Per ml IgG solution 50 µl Biotin -N-hydroxysuccinimide (3.6 mg/ml in DMSO) are added. After 30 min at room temperature, the sample is chromatographed on Superdex 200 (10 mM NaH₂PO₄/NaOH, pH 7.5, 30 mM NaCl). The fraction containing biotinylated IgG are collected. Monoclonal antibodies are biotinylated according to the same procedure.

### Digoxygenylation of polyclonal rabbit IgG

Polyclonal rabbit IgG is brought to 10 mg/ml in 10 mM NaH₂PO₄/NaOH, 30 mM NaCl, pH 7.5. Per ml IgG solution 50 µl digoxigenin-3-O-methylcarbonyl-ε-aminocaproic acid-N-hydroxysuccinimide ester (Roche Diagnostics, Mannheim, Germany, Cat. No. 1 333 054) (3.8 mg/ml in DMSO) are added. After 30 min at room temperature, the sample is chromatographed on Superdex^{®} 200 (10 mM NaH₂PO₄/NaOH, pH 7.5, 30 mM NaCl). The fractions containing digoxigenylated IgG are collected. Monoclonal antibodies are labeled with digoxigenin according to the same procedure.

### Example 3

### Western Blotting for the detection of PRN3/ILEU and PRN3 in human colorectal cancer tissue using polyclonal antibody as generated in Example 2.

Tissue lysates from tumor samples and healthy control samples were prepared as described in Example 1, "Tissue preparation".

SDS-PAGE and Western-Blotting were carried out using reagents and equipment of Invitrogen, Karlsruhe, Germany. For each tissue sample tested, 10 µg of tissue lysate were diluted in reducing NuPAGE^{®} (Invitrogen) SDS sample buffer and heated for 10 min at 95°C. Samples were run on 4-12% NuPAGE^{®} gels (Tris-Glycine) in the MES running buffer system. The gel-separated protein mixture was blotted onto nitrocellulose membranes using the Invitrogen XCell II^{™} Blot Module (Invitrogen) and the NuPAGE^{®} transfer buffer system. The membranes were washed 3 times in PBS/0.05% Tween-20 and blocked with Roti^{®}-Block blocking buffer (A151.1; Carl Roth GmbH, Karlsruhe, Germany) for 2 h. The primary antibody, polyclonal rabbit anti-IELU or polyclonal rabbit anti-PRN3 serum (generation described in Example 2), was diluted 1:10,000 in Roti^{®}-Block blocking buffer and incubated with the membrane for 1 h. The membranes were washed 6 times in PBS/0.05% Tween-20. The specifically bound primary rabbit antibody was labeled with a POD-conjugated polyclonal sheep anti-rabbit IgG antibody, diluted to 10 mU/ml in 0.5 x Roti^{®}-Block blocking buffer. After incubation for 1 hour, the membranes were washed 6 times in PBS/0.05% Tween-20. For detection of the bound POD-conjugated anti-rabbit antibody, the membrane was incubated with the Lumi-Light^{PLUS} Western Blotting Substrate (Order-No. 2015196, Roche Diagnostics GmbH, Mannheim, Germany) and exposed to an autoradiographic film.

### Example 4

### ELISA for the measurement of total PRN3 in human serum and plasma samples.

For detection of total PRN3 in human serum or plasma, a sandwich ELISA is developed. For capture and detection of the antigen, aliquots of the anti-PRN3 polyclonal antibody (see Example 2) are conjugated with biotin and digoxygenin, respectively.

Streptavidin-coated 96-well microtiter plates are incubated with 100 µl biotinylated anti-PRN3 polyclonal antibody for 60 min at 10 µg/ml in 10 mM phosphate, pH 7.4, 1% BSA, 0.9% NaCl and 0.1% Tween 20. After incubation, plates are washed three times with 0.9% NaCl, 0.1% Tween 20. Wells are then incubated for 2 h with either a serial dilution of the recombinant protein (see Example 2) as standard antigen or with diluted plasma samples from patients. After binding of PRN3, plates are washed three times with 0.9% NaCl, 0.1% Tween 20. For specific detection of bound total PRN3, wells are incubated with 100 µl of digoxygenylated anti-PRN3 polyclonal antibody for 60min at 10 µg/ml in 10 mM phosphate, pH 7.4, 1% BSA, 0.9% NaCl and 0.1% Tween 20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0.9% NaCl and 0.1% Tween 20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 5

### ROC analysis to assess clinical utility in terms of diagnostic accuracy.

Accuracy is assessed by analyzing individual liquid samples obtained from well-characterized patient cohorts, i.e., 50 patients having undergone colonoscopy and found to be free of adenoma or CRC, 50 patients diagnosed and staged as T1-3, N0, M0 of CRC, and 50 patients diagnosed with progressed CRC, having at least tumor infiltration in at least one proximal lymph node or more severe forms of metastasis, respectively. CEA as measured by a commercially available assay (Roche Diagnostics, CEA-assay (Cat No. 1 173 1629 for Elecsys^{®} Systems immunoassay analyzer) and total PRN3 measured as described above are quantified in a serum obtained from each of these individuals. ROC-analysis is performed according to Zweig, M. H., and Campbell, *supra*. Discriminatory power for differentiating patients in the group Tᵢₛ-3, N0, M0 from healthy individuals as measured by the area under the curve is found to be at least as good for total PRN3 as compared to the established marker CEA.

Preliminary data indicate that total PRN3 may also be very helpful in the follow-up of patients after surgery.

### Examples 6

### ELISA for the measurement of PRN3/ILEU in human serum and plasma samples.

For detection of PRN3/ILEU in human serum or plasma, a sandwich ELISA is developed. Anti-PRN3 polyclonal antibody and anti-ILEU polyclonal antibody are used for capture and detection, respectively, or vice versa. For capture of the antigen, aliquots of the anti-PRN3 polyclonal antibody or the anti-ILEU polyclonal antibody (see Example 2) are conjugated with biotin. For detection of the antigen, aliquots of the anti-PRN3 polyclonal antibody or the anti-ILEU polyclonal antibody (see Example 2) are conjugated with digoxygenin.

Streptavidin-coated 96-well microtiter plates are incubated with 100 µl biotinylated anti-PRN3 or anti-ILEU polyclonal antibody for 60 min at 10 µg/ml in 10 mM phosphate, pH 7.4, 1% BSA, 0.9% NaCl and 0.1% Tween 20. After incubation, plates are washed three times with 0.9% NaCl, 0.1% Tween 20. Wells are then incubated for 2 h with either a serial dilution of the recombinant protein (see Example 2) as standard antigen or with diluted plasma samples from patients. After binding of the target antigen PRN3 or ILEU, plates are washed three times with 0.9% NaCl, 0.1% Tween 20. For specific detection of bound PRN3/ILEU, wells are incubated with 100 µl of digoxygenylated anti-ILEU or anti-PRN3 polyclonal antibody for 60 min at 10 µg/ml in 10 mM phosphate, pH 7.4,1% BSA, 0.9% NaCl and 0.1% Tween 20. Thereafter, plates are washed three times to remove unbound antibody. In a next step, wells are incubated with 20 mU/ml anti-digoxigenin-POD conjugates (Roche Diagnostics GmbH, Mannheim, Germany, Catalog No. 1633716) for 60 min in 10 mM phosphate, pH 7.4, 1% BSA, 0.9% NaCl and 0.1% Tween 20. Plates are subsequently washed three times with the same buffer. For detection of antigen-antibody complexes, wells are incubated with 100 µl ABTS solution (Roche Diagnostics GmbH, Mannheim, Germany, Catalog No. 11685767) and OD is measured after 30-60 min at 405 nm with an ELISA reader.

### Example 7

### ROC analysis to assess clinical utility in terms of diagnostic accuracy.

Accuracy is assessed by analyzing individual liquid samples obtained from well-characterized patient cohorts, i.e., 50 patients having undergone colonoscopy and found to be free of adenoma or CRC, 50 patients diagnosed and staged as T1-3, N0, M0 of CRC, and 50 patients diagnosed with progressed CRC, having at least tumor infiltration in at least one proximal lymph node or more severe forms of metastasis, respectively. CEA as measured by a commercially available assay (Roche Diagnostics, CEA-assay (Cat. No. 1 173 1629 for Elecsys^{®} Systems immunoassay analyzer) and PRN3/ILEU measured as described above have been quantified in a serum obtained from each of these individuals. ROC-analysis is performed according to Zweig, M. H., and Campbell, *supra.* Discriminatory power for differentiating patients in the group T₁-3, N0, M0 from healthy individuals as measured by the area under the curve is found to be at least as good for PRN3/ILEU as compared to the established marker CEA.

Preliminary data indicate that PRN3/ILEU may also be very helpful in the follow-up of patients after surgery.

### List of References

Ahlquist, D.A., GastroenteroL Clin. North Am. 26 (1997) 41-55
Anderson, W.F., et al., J. Natl. Cancer Institute 94 (2002) 1126-1133
Baggiolini, M., et al., Agents Actions 8 (1978) 3-10
Bories, D., et al., Cell 59 (1989) 959-968
Bruck, C., et al., Methods Enzymol. 121 (1986) 587-695
Brünagel, G., et al., Cancer Research 62 (2002) 2437-2442
Carriquiry, L.A., and Pineyro, A., Dis. Colon Rectum 42 (1999) 921-929
Campanelli, D., et al., J. Clin. Invest 85 (1990) 904-915
Charles, L.A., et al., J. Leuk. Biol. 50 (1991) 539-546
Cooley, J., et al., Biochem. 40 (2001) 15762-15770
Csernok, E., et al., Advan. Exp. Med. Biol. 336 (1993) 45-50
Diamandis, et al., eds. (1996) Immunoassay, Academic Press, Boston.
Galfre, G., and Milstein, C., Methods Enzymol. 73 (1981) 3-46
Geenen, J.E., et al., Am. J. Dig. Dis. 20 (1975) 231-235
Goldenberg, D.M., et al., J. Natl. Cancer Inst. (Bethesda) 57 (1976) 11-22
Herrera, M.A., et al., Ann. Surg. 183 (1976) 5-9
Kao, R.C., et al., J. Clin. Invest. 82 (1988) 1963-1973
Ludemann, J., et al., J. Exp. Med.171 (1990) 357-362
Martell, R.E., et al., Int J. Biol. Markers 13 (1998) 145-149
Moertel, C.G., et al., JAMA 270 (1993) 943-947
Olson, S., et aL, JBC 270 (1995) 30007-30017
Remold-O'Donnell, E., et al., PNAS 89 (1992) 5635-5639
Reynoso, G., et al., JAMA 220 (1972) 361-365
Silvis, S.E., et al., JAMA 235 (1976) 928-930
Sobin, L.H., Wittekind, Ch. (eds), TNM Classification of Malignant Tumours, fifth edition, 1997
Studier, F.W., et al., Methods Enzymol. 185 (1990) 60-89
Sturgeon, C., Clinical Chemistry 48 (2002) 1151-1159
Tijssen, P., Practice and theory of enzyme immunoassays 11 (1990) the whole book, especially pages 43-78; Elsevier, Amsterdam
UICC (International Union Against Cancer), Sobin, L.H., Wittekind, Ch. (eds), TNM Classification of Malignant Tumours, fifth edition, 1997
Wanebo, H.J., et al., N. Engl. J. Med. 299 (1978) 448-451
Wilde, C.G., et al., J. Biol. Chem. 265 (1990) 2038-2041
WO 01/96390
WO 02/078636
Zweig, M. H., and Campbell, G., Clin. Chem. 39 (1993) 561-577

## Claims

1. A method for the diagnosis of colorectal cancer (=CRC) comprising the steps of
a) providing a liquid sample obtained from an individual,
b) contacting said sample with a first specific binding agent for proteinase 3 (PRN3) and a second specific binding agent for the serpin-type proteinase inhibitor leukocyte elastase inhibitor (ILEU) under conditions appropriate for formation of a complex between said first and second binding agent and PRN3 bound to ILEU (PRN3/ILEU), and
c) correlating the amount of complex formed in (b) to the diagnosis of colorectal cancer.

2. The method according to claim 1, further **characterized in that** said sample is serum.

3. The method according to claim 1, further **characterized in that** said sample is plasma.

4. The method according to claim 1, further **characterized in that** said sample is whole blood.

5. Use of PRN3/ILEU as a marker molecule in the diagnosis of colorectal cancer from a liquid sample obtained from an individual.

6. Use of PRN3/ILEU as a marker molecule in the early diagnosis of colorectal cancer from a liquid sample obtained from an individual.

7. Use according to claim 6, wherein the early diagnosis is made with sample derived from CRC patients in the adenoma stage.

8. Use according to claim 6, wherein the early diagnosis is made with sample derived from CRC patients in stage Tᵢₛ -3; N0; M0

9. Use of PRN3/ILEU as a marker molecule for colorectal cancer in combination with another marker molecule for colorectal cancer in the diagnosis of colorectal cancer from a liquid sample obtained from an individual.

## Patentansprüche

1. Verfahren zur Diagnose von Kolorektalkarzinom (CRC), bei dem man die folgenden Schritte durchführt:
a) Bereitstellen einer einem Individuum entnommenen Flüssigkeitsprobe,
b) Inkontaktbringen der Probe mit einem ersten spezifischen Bindungsmittel für Proteinase 3 (PRN3) und einem zweiten spezifischen Bindungsmittel für den Proteinase-Inhibitor vom Serpin-Typ, Leukozyten-Elastase-Inhibitor (ILEU), unter Bedingungen, die für die Ausbildung eines Komplexes zwischen dem ersten und dem zweiten Bindungsmittel und an ILEU (PRN3/ILEU) gebundener PRN3 geeignet sind, und
c) Korrelieren der Menge an in (b) gebildetem Komplex mit der Diagnose von Kolorektalkarzinom.

2. Verfahren nach Anspruch 1, ferner **dadurch gekennzeichnet, dass** es sich bei der Probe um Serum handelt.

3. Verfahren nach Anspruch 1, ferner **dadurch gekennzeichnet, dass** es sich bei der Probe um Plasma handelt.

4. Verfahren nach Anspruch 1, ferner **dadurch gekennzeichnet, dass** es sich bei der Probe um Vollblut handelt.

5. Verwendung von PRN3/ILEU als Markermolekül bei der Diagnose von Kolorektalkarzinom anhand einer einem Individuum entnommenen Flüssigkeitsprobe.

6. Verwendung von PRN3/ILEU als Markermolekül bei der Frühdiagnose von Kolorektalkarzinom anhand einer einem Individuum entnommenen Flüssigkeitsprobe.

7. Verwendung nach Anspruch 6, wobei die Frühdiagnose mit aus Patienten mit CRC im Adenomstadium gewonnener Probe durchgeführt wird.

8. Verwendung nach Anspruch 6, wobei die Frühdiagnose mit aus Patienten mit CRC im Stadium Tᵢₛ-3; N0; M0 gewonnener Probe durchgeführt wird.

9. Verwendung von PRN3/ILEU als Markermolekül für Kolorektalkarzinom in Kombination mit einem weiteren Markermolekül für Kolorektalkarzinom bei der Diagnose von Kolorektalkarzinom anhand einer einem Individuum entnommenen Flüssigkeitsprobe.

## Revendications

1. Procédé pour le diagnostic du cancer colorectal (= CRC), comprenant les étapes
a) de procuration d'un échantillon liquide prélevé d'un individu ;
b) de mise en contact dudit échantillon avec un premier agent de liaison spécifique pour la protéinase 3 (PRN3) et avec un deuxième agent de liaison spécifique pour l'inhibiteur de protéinase de type serpine inhibiteur de leucocyte élastase (ILEU) dans des conditions appropriées pour la formation d'un complexe entre ledit premier et ledit deuxième agent de liaison et PRN3 lié à ILEU (PRN3/ILEU) ; et
c) d'établissement d'une corrélation entre la quantité du complexe formé sous (b) et le diagnostic d'un cancer colorectal.

2. Procédé selon la revendication 1, **caractérisé en ce que** ledit échantillon est du sérum.

3. Procédé selon la revendication 1, **caractérisé en ce que** ledit échantillon est du plasma.

4. Procédé selon la revendication 1, **caractérisé en ce que** ledit échantillon est du sang total.

5. Utilisation de PRN3/ILEU comme molécule de marquage dans le diagnostic d'un cancer colorectal à partir d'un échantillon liquide prélevé d'un individu.

6. Utilisation de PRN3/ILEU comme molécule de marquage dans le diagnostic précoce d'un cancer colorectal à partir d'un échantillon liquide prélevé d'un individu.

7. Utilisation selon la revendication 6, dans laquelle le diagnostic précoce est établi avec un échantillon dérivé de patients CRC au stade d'adénome.

8. Utilisation selon la revendication 6, dans laquelle le diagnostic précoce est établi avec un échantillon dérivé de patients CRC au stade Tᵢₛ -3 ; N0 ; M0.

9. Utilisation de PRN3/ILEU comme molécule de marquage d'un cancer colorectal en combinaison avec d'autres molécules de marquage pour le cancer colorectal dans le diagnostic du cancer colorectal à partir d'un échantillon liquide prélevé d'un individu.
